# EUROPEAN PATENT SPECIFICATION

(11) **EP 2 332 903 B1**
(45) Date of publication and mention of the grant of the patent: **13.01.2016**
(21) Application number: 09810943.2
(22) Date of filing: 03.09.2009
(51) Int. Cl.: B01D 17/06, B03C 11/00, C07C 67/48, C10L 1/02, C11C 1/08, C11C 3/00

(54) **BIODIESEL PURIFICATION METHOD**
REINIGUNGSVERFAHREN FÜR BIODIESEL
PROCÉDÉ DE PURIFICATION D'UN BIODIESEL

(30) Priority: 04.09.2008 BR PI0804083
(43) Date of publication of application: 15.06.2011
(73) Proprietor: Petróleo Brasileiro S.A. - PETROBRAS, Rio de Janeiro, RJ 20035-900 (BR)
(72) Inventor: CARLOS NAGIB KHALIL, 21.931-300 Rio de Janeiro - RJ (BR); RODOLFO DE VICO, 24.240-000 Santa Rosa Niterói - RJ (BR); ANDRE MANOEL GOJA FERREIRA, 20.756-110 Rio de Janeiro - RJ (BR); BIANCA MACHADO DA SILVA FERREIRA, 22.750-290 Rio de Janeiro - RJ (BR)
(74) Representative: Duckworth, Timothy John
(86) International application number: PCT/BR2009/000273
(87) International publication number: WO 2010/025533

(56) References cited:
- EP-A1- 1 867 702
- BR-A- 0 105 888
- US-A1- 2008 202 021

## Description

### FIELD OF THE INVENTION

The present invention concerns a process for purification of biodiesel for the purpose of promoting the efficient separation of the glycerine fraction formed during transesterification of a mixture of long-chain triglycerides derived from vegetable and/or animal oils, with the aim of obtaining biodiesel (mixture of esters), in relatively short spaces of time, between the first and second transesterification reaction step, and, if necessary, also in the final product purification steps, the biodiesel fraction and the glycerine fraction.

More specifically, the present invention concerns the application of a method for electrostatic separation of the glycerine fraction to conventional processes for the industrial production of biodiesel.

### BASES OF THE INVENTION

For the industrial production of biodiesel - a renewable fuel to replace diesel from oil - the alkaline transesterification reaction of triglycerides, such as vegetable oils or even animal fats is commonly used, in the presence of a primary alcohol, in particular methanol and ethanol. The catalyst used, in a range of 0.5% to 1.5%, comes from hydroxides or alkoxides (methoxides and ethoxides) of alkaline metals, such as sodium and potassium. The commercial product known as biodiesel is chemically classified as a mixture of alquidic esters of fatty acids obtained by means of a transesterification reaction.

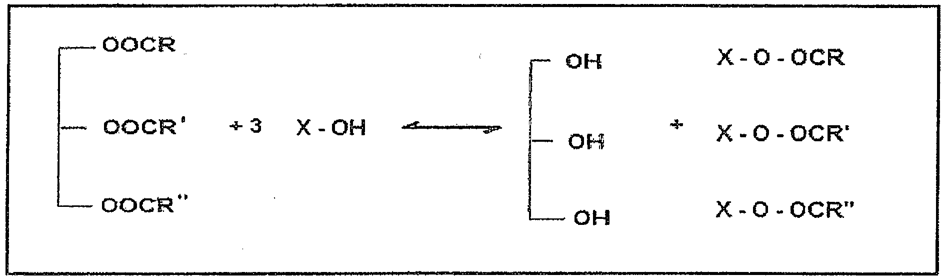

Nevertheless, the transesterification of triglycerides is a reversible reaction and its equilibrium is highly dependent upon the molar ratio between the reagents. Intermediate products of the alcoholysis reaction, such as mono- and diglycerides, are formed in varying concentrations, thereby establishing a complex equilibrium between the classes.

The excess alcohol may favour a shift in the reaction in the direction of the products, but it will be difficult to achieve full conversion from the reaction. On the other hand, both the use of an excess of alcohol in relation to the stochiometric equilibrium value of 1:3, and the generation of glycerine equivalent to 1 mol for each mol of vegetable oil, promote an undesirable stabilisation of the ternary mixture (esters \ alcohol \ glycerine) resulting from the primary step of the transesterification reaction.

One solution that can be adopted to achieve higher conversion factors is to perform the process in two steps, but to do this it is first necessary to remove the glycerine produced in the first step.

On the other hand, the full removal of the glycerine content generated in the first step requires relatively long dwell times in the decanting vessels. The inefficiency of the glycerine removal compromises execution of the second step of the transesterification reaction, and, consequently, reduces the quality of the final biodiesel and the overall yield from the process.

The present invention is aimed at the insertion and implementation of a stage of a process to promote the efficient separation of the glycerine fraction, in relatively short spaces of time between the first and second transesterification reaction step, and, if necessary, also in the final product purification steps, the biodiesel fraction and the glycerine fraction.

### RELATED METHOD

Brazilian patent application PI 0105888-6concerns a process for the production of biodiesel, which is obtained directly from oleaginous seeds rich in triglycerides, preferably castor seeds, by means of a transesterification reaction in which the seeds themselves are brought into a reaction with anhydrous ethanol in the presence of an alkaline catalyst, to generate ethyl esters and glycerine as a by-product. These esters are separated by decantation, neutralised and used as fuel for diesel motors, or in mixtures of diesel with gasoline or ethanol.

The glycerine fraction separation step in the industrial process for biodiesel production is conventionally handled as a separate operation of the gravitational or centrifugal decantation type, almost always preceded by the addition of percentages of water in a range of 5% to 20%.

The presence of water in the ternary system (esters \ alcohol \ glycerine) promotes a reduction in viscosity and an increase in polarity of the glycerine fraction, which are highly advantageous in its decantation under conditions of rest or centrifugation.

However, the presence of intermediate compounds from the transesterification reaction, such as mono- and diglycerides, or saponified compounds, originating from secondary reactions between the alkaline catalyst and the triglycerides, leads to the stabilisation of a persistent emulsified system. The stable emulsion contains the hydrated glycerine as a dispersed phase; the esters and mono- and diglyceride intermediate compounds as a dispersing phase; and the saponified compounds as emulsifying agents. The alcohol component remaining in the process is subdivided in proportion between the two phases.

A theoretical alternative in order to overcome the glycerine separation problem would be to reduce the alcohol content by distillation and subsequent neutralisation of the catalyst, with the addition of a mineral acid. Such steps would, however, eventually lead to other problems for the purification process, such as the partial reversal of the reaction - with the formation once again of the intermediate compounds (mono- and diglycerides) and the precipitation of fatty acids - due to the neutralisation of the saponified compounds. The point of equilibrium between these two steps is difficult to set and highly dependent upon the characteristics of the reaction charge.

Methanol, being more reactive than ethanol, is used more frequently in industrial processes. However, due to its toxicity, it is being replaced by ethanol which in addition to being less toxic can be obtained from a renewable source.

On the other hand, the type of catalyst used, the reaction conditions and the type and concentration of impurities in the reaction medium, determine the course of, the yield from and the by-products obtained in, the transesterification of the triglycerides,

One of these by-products of the reaction is the formation of soaps, which leads to stable emulsions. Under agitation, the glycerine disperses in the biodiesel in the form of minute droplets; with their surfactant action, the soap molecules deposit on the surface of the droplets giving rise to emulsion. For this reason, the step in which the glycerine is separated from the biodiesel constitutes a critical step in the industrial process.

The purification process now being proposed, aims to solve this problem by drastically reducing the time for separation of the phases.

The present invention concerns the application of an electrostatic separation method to the conventional processes for industrial production of biodiesel.

EP 1 867 702 mentions a method for purifying biodiesel fuel comprising the application of an electric field or heating.

### SUMMARY OF THE INVENTION

The present invention concerns a process for purification of biodiesel obtained from castor seed oil, for the purpose of promoting the efficient separation of the glycerine fraction formed during the transesterification reaction of a mixture of long-chain triglycerides derived from the oil in the presence of ethanol and an alkaline catalyst.

The invention concerns the application of the electrostatic separation method to biodiesel from castor oil in order to separate the glycerine from the stable emulsion then formed, in relatively short spaces of time.

The biodiesel purification process that is the subject matter of the present invention comprises the steps of:
- partially removing by evaporation the excess ethanol present in the mixture of esters which is composed of 60% - 80% of alkydic esters, 5% - 15 % of glycerine, and 15% - 30 % of ethanol, using a heating bath to maintain the mixture at a temperature of approximately 90°C;
- adding to the mixture resulting from the evaporation of the ethanol approximately 0.7% water and 2% - 5% n-hexane, whilst homogenising it;
- transferring the homogenised mixture to a decanting vessel provided with electrodes, promoting contact between said mixture and a glycerine stock existing in the decanting vessel, in a proportion of 10:1 - 5:1 by volume;
- applying an alternating electrical current, provided by the operation of high voltage converters, in the range 1 - 5 KV, in order to promote the electrostatic separation of the glycerine.

### DETAILED DESCRIPTION OF THE INVENTION

The present invention concerns the application of an electrostatic separation method, known as electrocoalescence, in order to separate the glycerine fraction. The phenomenon can be explained in the following way:
Let us take as an example the separation of water dispersed in petroleum. The petroleum, being of an organic nature, has a low electrical conductivity. For their part, the salts dispersed in the water give the dispersed phase a high electrical conductivity. When a droplet of water has a high intensity alternating current field applied to it, the drop of water elongates and dipoles are generated with an electrical charge in opposing directions. Another droplet that is located in the vicinity of the polarised droplet will also undergo polarisation but, through an inductive effect, its dipoles will have opposing electrical charges. The electrical interaction between the dipoles with opposing charges means that the two droplets are attracted, leading to an impact between and coalescence of these droplets.

In the case in question the biodiesel, a dispersing phase and organic in nature, has low electrical conductivity, while the glycerine, a dispersed phase, has high electrical conductivity, principally due to being in the presence of ions of the alkaline catalyst in the solution.

Following the first step of the transesterification of triglycerides reaction, in alkaline and necessarily anhydrous medium, the conversion factor is less than 90% expressed in moles of ester. Removal of the glycerine content is essential in this case in order for the second step of the reaction to take place.

In the specific case of the use of castor seed oil (which is highly hydroxylated) and ethanol, the situation is aggravated making the separation of the glycerine (also rich in hydroxyls) difficult due to the formation of a stable system, with high involvement of polar forces, which calls for decantation times of up to 24 hours or more in order for the separation of the glycerine to take place. It is known that excess alcohol in the reaction medium is a critical factor in the separation of glycerine from the biodiesel.

Laboratory tests have proven that the proper application of an electrical field of high voltage and low amperage within the liquid mixture resulting from the first step promotes a rapid separation of the dispersed (polar) and dispersing (apolar) phase.

The electrical field initially promotes the polarisation of the droplets of the glycerine phase (polar). A sequentially polarised ordering of the droplets takes place immediately in the dispersed phase when subjected to the electrical field. After reaching a minimum level of agglomeration the drops undergo rapid drainage of their interstitial film - comprising a thin layer of liquid from the external phase (esters). In this way larger drops form and finally the process advances through the coalescence of the drops followed by sedimentation through purely gravitational action.

### DESCRIPTION OF THE PREFERRED EMBODIMENT

In order to provide a better understanding of the invention, it will be explained using examples of a merely illustrative nature and which do not constitute any limitation to its practical application. It will be obvious to a person skilled in the art that other embodiments may be used without deviating from the inventive concept presented here.

### Example 1: Applicability of the electrostatic separation method to biodiesel.

Initially the tests were performed in the laboratory, using apparatus comprising an electrical voltage transformer, and a graduated tube with a capacity of 100 ml containing the electrode, which was connected to a circulating heating bath at a temperature of 60°C.

Tests were carried out using liquors - the name given to the system resulting from the biodiesel production reaction - obtained from various vegetable oils, such as for example, sunflower, corn, canola, soya and castor seeds, by the transesterification reaction of the oil with ethanol, in the presence of sodium hydroxide **(NaOH)** as the catalyst. The liquors which also contained in dispersed form the glycerine phase of the ester phase, were agitated, transferred to the graduated tube and the electric voltage was immediately applied with the separation of the glycerine being observed over time.

For the purposes of comparison the glycerine separation was also quantified using the traditional method that is to say by gravitational segregation without use of an electrical field. The results are presented in Table 1 below.

| **TABLE 1** | | | | |
|---|---|---|---|---|
| **Liquor** | **Voltage (V)** | **Current (mA)** | **Separation time** | **Efficiency (%)** |
| Sunflower | 1400 | 14.0 | 17s | 100 |
| | 1000 | 7.5 | 28 s | 100 |
| | 900 | 5.0 | 29 s | 100 |
| | 0 | 0 | 6 min | 100 |
| Corn | 800 | 15.0 | 20 s | 100 |
| | 1000 | 6.0 | 22 s | 100 |
| | 900 | 4.0 | 28 s | 100 |
| | 0 | 0 | 6 min | 100 |
| Canola | 1500 | 14.0 | 15 s | 100 |
| | 1000 | 5.0 | 20s | 100 |
| | 700 | 4.0 | 21 s | 100 |
| | 0 | 0 | 6 min | 100 |
| Soya | 2700 | 14.0 | 17 s | 100 |
| | 1400 | 3.0 | 19 s | 100 |
| | 900 | 5.0 | 27 s | 100 |
| | 0 | 0 | 6 min | 100 |
| Castor seeds | 2600 | 8.0 | - | 0 |

As can be seen, there was a rapid separation of the glycerine in the majority of cases, when the liquors were subjected to the electrical field with the exception of the castor seed oil; the performance of the separation for these oils tested was fairly similar. The increase in intensity of the electrical field was accompanied by a reduction in the separation time and, in addition, this time was much less than the time necessary for separation to take place by gravitation.

### Example 2: Effect of the presence of ethanol on the separation.

The performance of the electrostatic separation of the glycerine from the biodiesel, when in the presence of an excess of alcohol was observed by adding ethanol to the liquors of the above example.

The results confirm that the excess ethanol provides the system with high stability and can even prevent separation, as shown in Table 2.

| **TABLE 2** | | | | | |
|---|---|---|---|---|---|
| **Liquor** | **Ethanol (%)** | **Voltage (V)** | **Current (mA)** | **Separation time (s)** | **Efficiency (%)** |
| Sunflower | 2.5 | 500 | 8.0 | - | 0 |
| Corn | 2.5 | 1500 | 14.0 | - | 0 |
| Canola | 2.5 | 2700 | 6.0 | - | 0 |
| Soya | 2.5 | 900 | 16.0 | 59 | 100 |
| | | 600 | 8.0 | 90 | 100 |
| | | 400 | 4.0 | 120 | 100 |
| | 5.0 | 1500 | 14.0 | - | 0 |

### Example 3: Applicability to castor oil.

Since the separation of the glycerine phase was not observed when liquor was used obtained from castor seeds, new studies were conducted with the aim of shedding light on this matter.

So, part of the excess of ethanol was removed from the liquor obtained from castor seeds, with the aim of promoting the destabilisation of the dispersion, by means of evaporation, using a heating bath temperature of approximately 90°C. Ethanol content reductions with values of 5%, 10% and 15% were tested.

In order to increase the difference in polarity between the phases, n-hexane was added to the liquor in proportions of 2% and 5%, and water in a proportion of 0.7%. The results are presented below in Table 3.

It was found that the removal of part of the ethanol from the liquor obtained from the castor seeds allowed the method to be applied and, consequently, the separation of the glycerine which previously did not happen.

Moreover, the increased removal of the ethanol was accompanied by an increased separation of the glycerine (with the removal of 15% of the ethanol separation took place in relatively short spaces of time).

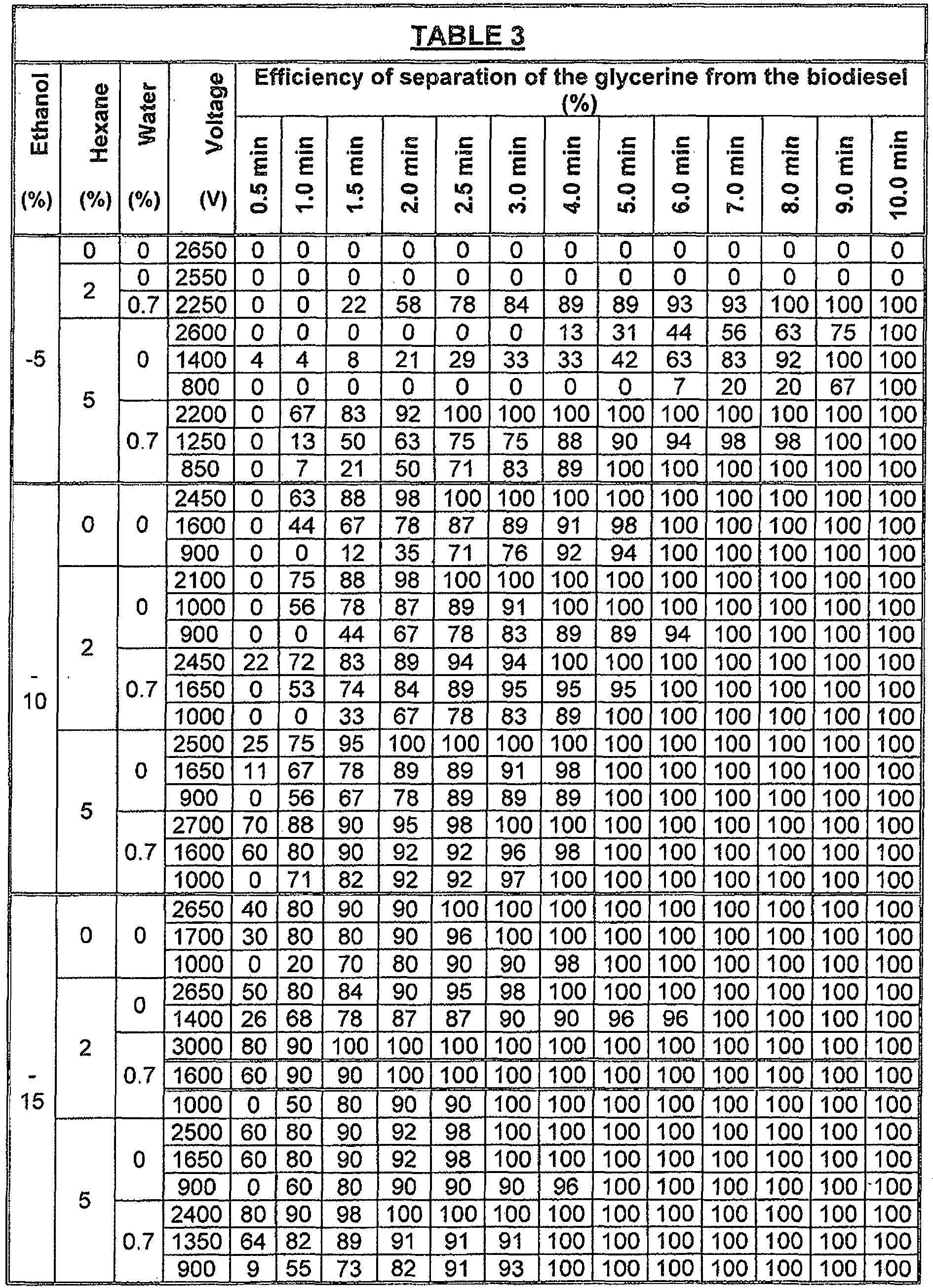

### Example 4: Specific example of the invention.

Bearing in mind that the aim of the invention is to obtain biodiesel from castor seed oil, a practical application on a pilot scale of this invention is the installation of electrodes, powered by high voltage transformers (1 - 5 KV) in horizontal decanting vessels, originally intended for the gravitational separation of the glycerine phase contained in the ternary system removed from the transesterification reactor.

The lower part of the decanting vessel contains up to 50% of the total volume of the vessel by glycerine volume (stock). The liquor originating from the reactor has a composition of 60% - 80% alkydic esters (biodiesel), 5% - 15 % glycerol (by-product), and 15% - 30 % alcohol (non-reacted excess). The passing of this liquor as a continuous flow through this layer of glycerine in a proportion of up to 10:1 by volume promotes saturation of the system.

The saturated mixture is subjected to the electric field which promotes the polarisation, coalescence and decantation steps of the disperse phase (glycerine). Bearing in mind that the catalyst used also has a polar characteristic, this is decanted together with the glycerine phase.

At the same a mixture of polar \ apolar liquid pairings is used, for example water and n-hexane, in suitable proportions, in order to provide greater differentiation of the polarity indices between the esters fraction and the glycerine fraction. The combination of the two effects favours the destabilisation of systems that are highly resistant to conventional separation by simple decantation.

It is in this system group that the biodiesel produced from castor oil is found, which is known to be present difficulties with the separation of phases due to the presence of a hydroxyl grouping (OH) in the 12^{th} carbon in a chain of 18 ricinoleic acid atoms.

It was found that the prior addition of n-hexane and water in a ratio of 5:1 by volume to the ternary system of the biodiesel from castor oil, followed by the application of alternating current **(AC)** of the order of 2 KV strongly favour the separation of the dispersed and dispersing phases, in a time of the order of up to 10 seconds, depending on the operational conditions employed.

## Claims

1. **PROCESS FOR PURIFICATION OF BIODIESEL**, obtained from the transesterification of castor seed oil in the presence of ethanol and an alkaline catalyst **characterised by** separating the glycerine formed during the transesterification reaction, comprising the steps of:
- partially removing by evaporation the excess ethanol present in the mixture of esters which is composed of 60% - 80% of alkydic esters, 5% - 15 % of glycerine, and 15% - 30 % of ethanol, using a heating bath to keep the mixture at a temperature of approximately 90 °C;
- adding to the mixture resulting from the evaporation of the ethanol approximately 0.7% water and 2% - 5% n-hexane, whilst homogenising it;
- transferring the homogenised mixture to a decanting vessel provided with electrodes, promoting contact between said mixture and a glycerine stock existing in the decanting vessel, in a proportion of 10:1 - 5:1 by volume;
- applying an alternating electrical current, provided by the operation of high voltage converters, in the range 1 - 5 KV, in order to promote the electrostatic separation of the glycerine.

2. **PROCESS FOR PURIFICATION OF BIODIESEL**, according to claim 1, **characterised by** reducing the stability of the emulsion formed by the esters / ethanol / glycerine system, by means of the application of a high voltage electric field.

3. **PROCESS FOR PURIFICATION OF BIODIESEL**, according to claim 1, **characterised by** removal of between 5% - 15% by volume of ethanol from the mixture of esters.

## Patentansprüche

1. Verfahren zur Reinigung von Biodiesel, der durch Umesterung von Rizinusöl in Gegenwart von Ethanol und einem alkalischen Katalysator gewonnen wird, **gekennzeichnet durch** das Abscheiden von während der Umesterungsreaktion entstandenem Glycerin, umfassend die folgenden Schritte:
- teilweises Entfernen des in dem Gemisch aus Estern vorhandenen überschüssigen Ethanols **durch** Verdampfung, wobei das Gemisch aus Estern aus 60 % - 80 % Alkydestern (engl.: *alhydic esters),* 5 % - 15 % Glycerin und 15 % - 30 % Ethanol zusammengesetzt ist, unter Verwendung eines Heizbades, um das Gemisch auf einer Temperatur von ungefähr 90 °C zu halten;
- Zugeben von ungefähr 0,7 % Wasser und 2 % - 5 % n-Hexan zu dem aus der Verdampfung des Ethanols resultierenden Gemisch unter Homogenisierung desselben;
- Umfüllen des homogenisierten Gemischs in ein mit Elektroden versehenes Dekantiergefäß, Inkontaktbringen des Gemischs mit bereits in dem Dekantiergefäß vorhandenem Vorrat an Glycerin in einem Volumenverhältnis von 10:1 - 5:1;
- Anlegen eines **durch** den Betrieb von Hochspannungswandlern bereitgestellten Wechselstroms von 1 - 5 kV zur Förderung der elektrostatischen Abscheidung des Glycerins.

2. Verfahren zur Reinigung von Biodiesel nach Anspruch 1,
**gekennzeichnet durch** das Reduzieren der Stabilität der von dem System aus Estern/Ethanol/Glycerin gebildeten Emulsion mittels Anlegung eines elektrischen Hochspannungsfelds.

3. Verfahren zur Reinigung von Biodiesel nach Anspruch 1,
**gekennzeichnet durch** das Entfernen von 5 - 15 Volumen-% Ethanol aus dem Gemisch aus Estern.

## Revendications

1. Procédé pour la purification de biodiésel obtenu par transestérification d'huile de ricin en présence d'éthanol et d'un catalyseur alcalin, **caractérisé par** la séparation de la glycérine formée durant la réaction de transestérification, comprenant les étapes consistant à :
- éliminer partiellement par évaporation l'excès d'éthanol présent dans le mélange d'esters qui est composé de 60 % à 80 % d'esters alkydiques, de 5 % à 15 % de glycérine, et de 15 % à 30 % d'éthanol, par utilisation d'un bain chauffant pour maintenir le mélange à une température d'environ 90°C ;
- ajouter au mélange résultant de l'évaporation de l'éthanol environ 0,7 % d'eau et 2 % à 5 % de n-hexane, tout en l'homogénéisant ;
- transférer le mélange homogénéisé dans une cuve de décantation dotée d'électrodes, en favorisant le contact entre ledit mélange et un stock de glycérine existant dans la cuve de décantation, en une proportion de 10/1 à 5/1 en volume ;
- appliquer un courant électrique alternatif assuré par le fonctionnement de convertisseurs haute tension, dans la plage allant de 1 à 5 kV, afin de favoriser la séparation électrostatique de la glycérine.

2. Procédé pour la purification de biodiésel selon la revendication 1, **caractérisé par** une réduction de la stabilité de l'émulsion formée par le système esters/ éthanol/glycérine, au moyen de l'application d'un champ électrique haute tension.

3. Procédé pour la purification de biodiésel selon la revendication 1, **caractérisé par** l'élimination d'entre 5 % et 15 % en volume d'éthanol dans le mélange d'esters.
